# EUROPEAN PATENT SPECIFICATION

(11) **EP 1 407 759 B1**
(45) Date of publication and mention of the grant of the patent: **22.07.2009**
(21) Application number: 02022761.7
(22) Date of filing: 11.10.2002
(51) Int. Cl.: A61Q 19/00, A61K 8/73

(54) **A cosmetic composition in colloidal form comprising hyaluronic acids and chitosan**
Kosmetische Zubereitung in kolloidaler Form Hyaluronsäuren und Chitosan enthaltend
Composition cosmétique comprenant des acides hyaluronique et du chitosane

(43) Date of publication of application: 14.04.2004
(73) Proprietor: Zummer Ventures LLC, Morristown, NJ 08002 (US)
(72) Inventor: Giuseppe Petrignii, 20122 Milano (IT)
(74) Representative: Beneduce, Gianna

(56) References cited:
- EP-A- 0 735 049
- US-A- 5 928 653
- PATENT ABSTRACTS OF JAPAN vol. 014, no. 311 (M-0994), 4 July 1990 (1990-07-04) & JP 02 102008 A (SEIKO EPSON CORP;OTHERS: 01), 13 April 1990 (1990-04-13)
- PATENT ABSTRACTS OF JAPAN vol. 1999, no. 05, 31 May 1999 (1999-05-31) & JP 11 043425 A (NIPPON BIO KK), 16 February 1999 (1999-02-16)
- PATENT ABSTRACTS OF JAPAN vol. 013, no. 487 (C-649), 6 November 1989 (1989-11-06) & JP 01 190614 A (KANEBO LTD), 31 July 1989 (1989-07-31)
- PATENT ABSTRACTS OF JAPAN vol. 017, no. 131 (C-1036), 18 March 1993 (1993-03-18) & JP 04 305517 A (KANEBO LTD), 28 October 1992 (1992-10-28)
- PATENT ABSTRACTS OF JAPAN vol. 015, no. 076 (C-0809), 21 February 1991 (1991-02-21) & JP 02 300109 A (SANKO SEIBUTSU KAGAKU KENKYUSHO:KK), 12 December 1990 (1990-12-12)

## Description

The present invention relates to a cosmetic, colloidal composition having a strong moisturizing activity and useful for helping the elasticity of the skin.

The cosmetic composition consists of a colloidal solution containing, as active ingredients, a suitable mixture of hyaluronic acids of different molecular weights and chitosan, which are conveniently treated with one or more cosmetically acceptable diluents and preservatives.

A further object of the present invention is represented by the process used for preparing the cosmetic, colloidal composition of the present invention which contains, as active ingredients, a suitable mixture of different molecular weight hyaluronic acids and chitosan suitably treated with one or more proper diluents and preservatives which are cosmetologically acceptable and compatible with said active ingredients.

Hyaluronic acids contained in the cosmetic composition of the invention have a molecular weight varying from 400 to 4.000 daltons and the concentration of their mixture is at least of 5% in weight compared to the total weight of the cosmetic composition. More precisely, this concentration is comprised in amounts varying between 5 and 25% by weight compared to the total weight of the cosmetic composition and, more preferably, in quantities varying between 8 and 15% in weight compared to the total weight of the cosmetic composition.

Chitosan is present in the composition in an amount comprised between 0,5 and 2,5% in weight compared to the total weight of the cosmetic composition.

The amounts of chitosan and hyaluronic acids are included in the cosmetic composition of the invention in a ratio of weight of 1:10.

Examples of suitable diluents used in the cosmetic composition of the invention are distilled water, a saline solution, dimethylsulphoxide solutions or water solutions.

Examples of suitable preservative substances utilized in the cosmetic composition of the invention are p-hydroxybenzoic acid esters.

The cosmetic, colloidal composition object of the present invention has shown to be particularly effective in reducing the degenerative processes of the skin caused by the ageing or by the exposure to unfavourable environmental factors: the most evident characteristics of such degenerative processes are dehydration, dryness, loss of elasticity and the appearance of more or less marked wrinkles.

Hyaluronic acid is a mucopolysaccharide present in practically every part of living organisms, its distribution being almost ubiquitous in tissues and in organ parenchyma. The skin of an adult human organism, where there are about 25mg of hyaluronic acid per gram of weight in the epiderma and about 118mg of hyaluronic acid per gram of weight in the derma, is the largest seat of accumulation.

Hyaluronic acid is a saccharidic biopolymer, which forms by polymerisation of disaccharide units composed by an hexosamine (glucosamine) and by an esuronic (D-glucuronic) acid linked each other with glucosidic bonds; its molecular weight can vary from thousands to millions of daltons. Contrary to the other mucopolysaccharide substances, hyaluronic acid contains no sulphate groups, and is thought to possess, amongst all the glucosaminglicans, the simplest chemical structure.

Hyaluronic acid has the capacity to bond and retain large amounts of water in the interfibrillar spaces, up to 6 litres of water per gram, thus forming the backbone of the amorphous colloidal matrix acting as cement between cells and connective fibres, with relevant effects on microcirculatory exchanges thanks to its influence on interstitial volume, on water conductivity and on molecule diffusion.

Within the skin, solutions of hyaluronic acid in water give rise to gels that act as dampers.

Hyaluronic acid plays an important role in the organism in general and in particular in the skin, because with its capacity of moisturizing, softening, solute transport, cell growth control and cell differentiation, it succeeds in maintaining the skin moisturized, turgid, elastic, shining, young and in a physical gelatinous condition.

Hyaluronic acid represents the greatest responsible of the water-content of the tissues so that the quantity of water of the skin may be calculated from its content in hyaluronic acid.

Solutions of hyaluronic acid are characterized by being viscoelastic and pseudoplastic. The viscoelastic property of hyluronic acid, important as to its use as a biomaterial, is controlled by the concentration and by the molecular weight of its chains.

In cases of exposure to unfavourable environmental conditions or, specially, in getting on in years, the quantity of hyaluronic acid in the organism decreases and it can result less polymerized; as a consequence the quantity of water of retention decreases and the skin suffers alterations of its physical condition; an involutional process is clear in the epidermis with a general reduction and loss of elasticity due to a decrease of the degree of proliferation of the cells of epidermis, in the derm, with a reduced turnover of the cells and of the collagen and with, as a consequence, increasing of the reticular and fibrous structure which causes a wrinkling and a loss of elasticity of the skin.

Are known in the art cosmetic compositions wherein hyaluronic acid is contained: among them are those cited in Japanese Patent pubblication No.11043425 in the name of Nippon Bio KK which refers to an external preparation for skin damaged by sunburn containing chitosan and hyaluronic acid together with other active ingredients, all showing synergistic effects; and in Japanese Patent pubblication No.01190614 in the name of Kanebo Ltd. which refers to a skin cosmetic having protective effect on the skin and water holding effect and contains, together with other active ingredients, a mixture of a high molecular weight hyaluronic acid (PM≥1000000) and a low molecular weight hyaluronic acid (PM≤100000).

Document JP2101008 discloses a skin cosmetic comprising dutosan and hyaluronic acid

Hyaluronic acid may be extracted, as it was traditionally done up to a short time ago, from natural deposits, such as cockscomb, or from bovine connective tissues by separation methods consisting of an enzyme digestion, a specific separation intended to remove the proteins and a purification to obtain the crude extract.

These methods present several disadvantages connected to high production costs, poor control over molecular weight, and risk of viral infections.

Hyaluronic acid may also be produced by a biotechnological process, such as the bio-fermentation of gram-positive bacteria: this technique provides bio-polymers of hyaluronic acid without the mentioned drawbacks and in potentially unlimited quantities.

Chitosan is a complex biopolymer polysaccaride having a positive charge, which may be obtained from chitin by deacylation: chitin may be extracted from the exodermis of some marine crustaceans such as crabs, prawns, lobsters, and from zooplankton. While chitin is water insoluble, chitosan is soluble in water which makes it particularly convenient for its use.

In the skin, chitosan mixed with hyaluronic acid in specific weight ratios, preferably in a weight ratio chitosan:hyaluronic acid 1:10, improves hyaluronic acid diffusion increasing its activity and function so resulting in an improvement of the skin moisture and elasticity.

According to the process for the production of the cosmetic composition of the invention, solutions of hyaluronic acids having different molecular weight, comprised in the range from 400 to 4,000 daltons, are prepared.

The process of preparation is essentially based on the condensation of hyaluronic acid and a solution of hydrolyzed chitin (85-90% acetylglucosamine on the dry product) in the presence of a biological catalyst, at a temperature ranging from 18 to 20°C. A mixture of 10% aqueous solution hyaluronic acids in the form of transparent colloidal fluid, is obtained.

In a 100L steel turboreactor provided with an hollow space, 880g demineralized water are placed. The temperature of the melter is brought to 18-20°C and the pressure made under vacuum. Stirrer is activated at low speed and the temperature maintained at 18-20°C, then 100g of the mixture containing different molecular weight hyaluronic acids, 10g chitosan and 10g parabenzoic acid methyl ester are slowly added thereto. The mass obtained is homogenized at low speed for about 5 minutes, and when the product is completely homogenized, the stirrer is kept on at a low speed for a further 30 minutes. The product obtained is kept under vacuum for 60 minutes, then it is discharged from the termoemulsifier into a plastic container suitably covered with a proper polyethylene sterile bag and then tied up with a safety seal and stored.

The viscosity (h) and the elasticity (G) of the cosmetic composition of the invention related to the temperature have been evaluated. No correlation between temperature and elasticity was shown while an inverted correlation is evident between viscosity and temperature bearing in mind that the composition is prepared at 18-20°C.

It has been also evaluated the value of the spinnability of the composition.

### Viscosity (h)

| | | |
|---|---|---|
| At 5°C | h(mPas) | 7200.20 (immediate) |
| | h(mPas) | 6446.77 (after 5 minutes) |
| at 21°C | h(mPas) | 6001.79 (immediate) |
| | h(mPas) | 5931.37 (after 5 minutes) |
| at 37°C | h(mPas) | 5676.34 (immediate) |
| | h(mPas) | 5642.63 (after 5 minutes) |

### Spinnability

At room temperature = 18mm.

The cosmetic composition in colloidal form object of the invention has been tested, for a period of 30 days, on 20 women aged between 38 and 56.

Five women had never undergone any cosmetic treatment; four women had used moisturizing creams and 8% glycolic acid for about 2-3 months prior to the test; the remaining 11 women still used moisturizing creams and fruit acid derivatives: of these last women, four used lactic acid and seven used 8% glycolic acid. None of them have ever used for local cutaneous use, any hyaluronic acid based product and/or chitosan.

A further group of ten women had used as comparison a cosmetic product in colloidal form, which contained only a mixture of hyaluronic acids having the same molecular weight and the same concentration of the acids present in the mixture. All the cosmetic treatments lasted 30 days. Twenty women had applied to the face the cosmetic composition of the invention in the morning and evening; ten women had applied to the face, in the morning and evening, a cosmetic composition containing only hyaluronic acid having a concentration comprised between 8 and 15% by weight compared to the total weight of the composition.

All the thirty women showed a clear improvement of the skin which appeared, already after ten day treatment, less dry, less wrinkled, more moisturized, more elastic and radiant compared to the skin before the treatment.

The improvement in the skin was much more evident among the twenty women who had used the cosmetic colloidal composition containing the mixture of hyaluronic acids and chitosan in comparison to the effect obtained by the ten women who had used the colloidal composition containing only the mixture of hyaluronic acids.

## Claims

1. A cosmetic colloidal composition having a moisturizing activity and useful for helping the elasticity of the skin, **characterized by** that it contains a mixture of hyaluronic acids having different molecular weights comprised in the range from 400 to 4,000 dalton in an amount between 5 and 25% by weight compared to the total weight of the composition and an amount of chitosan comprised between 0.5 and 2.5% by weight compared to the total weight of the cosmetic composition and cosmetically acceptable diluents and preservatives wherein the chitosan and the mixture of hyaluronic acids are in a weight ratio of 1:10.

2. The cosmetic composition according to claim 1, **characterized by** that the weight of the hyaluronic acid mixture compared to the total weight of the cosmetic mixture is comprised between 8 and 15%.

## Patentansprüche

1. Eine kosmetische, kolloidale Zusammensetzung mit feuchtigkeitsspendender Wirkung und verwendbar zur Unterstützung der Elastizität der Haut, **dadurch gekennzeichnet, dass** sie eine Mischung von Hyaluronsäuren mit unterschiedlichen Molekulargewichten im Bereich von 400 bis 4000 Dalton, in einer Menge zwischen 5 und 25 Gew.-% im Vergleich zum Gesamtgewicht der Zusammensetzung enthält, und eine Menge an Chitosan im Bereich zwischen 0,5 und 2,5 Gew.-% im Vergleich zum Gesamtgewicht der kosmetischen Zusammensetzung und kosmetisch verträglicher Verdünnungsmittel und Konservierungsmittel, wobei das Chitosan und die Mischung der Hyaluronsäuren in einem Gewichtsverhältnis von 1:10 vorliegen.

2. Die kosmetische Zusammensetzung nach Anspruch 1, **dadurch gekennzeichnet, dass** das Gewicht der Hyaluronsäuremischung im Vergleich zum Gesamtgewicht der kosmetischen Mischung im Bereich zwischen 8 und 15 % liegt.

## Revendications

1. Composition colloïdale cosmétique ayant une activité hydratante et utile pour favoriser l'élasticité de la peau, **caractérisée en ce qu'**elle contient un mélange d'acides hyaluroniques ayant différentes masses moléculaires comprises dans la plage allant de 400 à 4000 daltons en une quantité de 5 à 25 % en poids par comparaison au poids total de la composition et une quantité de chitosane comprise dans la plage allant de 0,5 à 2,5 % en poids par rapport au poids total de la composition cosmétique et des diluants et des conservateurs acceptables sur le plan cosmétique, dans laquelle le chitosane et le mélange d'acides hyaluroniques sont dans un rapport en poids de 1/10.

2. Composition cosmétique selon la revendication 1, **caractérisée en ce que** le poids du mélange d'acides hyaluroniques par comparaison au poids total du mélange cosmétique est compris dans la plage allant de 8 à 15%.
